# EUROPEAN PATENT APPLICATION

(11) **EP 4 752 849 A1**
(43) Date of publication of application: **03.06.2026**
(21) Application number: 25218201.9
(22) Date of filing: 25.11.2025
(51) Int. Cl.: G06V 10/774, G01N 15/1433, G06V 20/69

(54) **APPARATUS, SYSTEM, METHOD, AND PROGRAM FOR EXTRACTING MICROBIAL IMAGE**

(30) Priority: 28.11.2024 JP 2024207720
(71) Applicant: Yokogawa Electric Corporation, Musashino-shi, Tokyo 180-8750 (JP)
(72) Inventor: Nakanishi, Hirofumi, Musashino-shi, Tokyo 180-8750 (JP)
(74) Representative: Hoffmann Eitle

(57) **Abstract**

(Solution) There is provides an apparatus including a processor, wherein the processor receives a plurality of captured images capturing a flow channel through which a microbe flows, and extracts a plurality of microbial images capturing a same individual of the microbe from the plurality of captured images. In the above-described apparatus, the flow channel may include a vortex generator which generates a vortex in an image-capturing area in the flow channel, in which the plurality of captured images are captured. In any of the above-described apparatus, the processor may receive the plurality of captured images which capture the flow channel at different timings.

## Description

### BACKGROUND

### 1. TECHNICAL FIELD

The present disclosure relates to an apparatus, system, method, and program for extracting a microbial image.

### 2. RELATED ART

Patent Document 1 describes that, in paragraph 0036, "the computer causes a generic object detection algorithm to learn images with a first predetermined size of rectangular shape, at the center of which a bounding box, or bbox, is located, as learning data", and that, in paragraph 0044, "160 images with a size of 2992 × 2992 px ... are visually checked."

### RELATED ART DOCUMENTS

### Patent document

Patent Document 1: Japanese Patent Application Publication No. 2021-158995

### SUMMARY

(1) In a first aspect of the present disclosure, there is provided an apparatus including a processor, wherein the processor receives a plurality of captured images capturing a flow channel through which a microbe flows, and extracts a plurality of microbial images capturing a same individual of the microbe from the plurality of captured images. The processor may identify whether the microbe contained in each microbial region is the same individual, based on at least one of a positional relationship or similarity among microbial regions in each of the plurality of captured images. The processor may extract the plurality of microbial images in which the same individual microbe is captured, for each of the plurality of microbes.
(2) In the apparatus described above in (1), the flow channel may include a vortex generator which generates a vortex in an image-capturing area in the flow channel, in which the plurality of captured images are captured. The vortex generator may be positioned in a fluid flowing through the flow channel.
(3) In the apparatus described above in (1) or (2), the processor may receive, in receiving the plurality of captured images, the plurality of captured images which capture the flow channel at different timings.
(4) In any of the apparatus described above in (1) to (3), the processor may receive, in the receiving the plurality of captured images, the plurality of captured images in which the flow channel is captured from two or more different directions.
(5) In any of the apparatus described above in (1) to (4), the processor may further generate learning data in which a specified label is added to the plurality of microbial images.
(6) In the apparatus described above in (5), the processor may further perform a process to generate an estimation model through learning, which estimates a species of the microbe from the microbial images, using the learning data.
(7) In any of the apparatus described above in (1) to (6), the processor may use each of the plurality of microbial images to further estimate a species of the microbe.
(8) In the apparatus described above in (7), the processor may further identify the species of the microbe, based on an estimation result regarding the species of the microbe estimated by using each of the plurality of microbial images.
(9) In a second aspect of the present disclosure, there is provided a system including any of the apparatus described above in (1) to (8); the flow channel; and an image-capturing unit which captures the plurality of captured images.
(10) The system described above in (9) may further include a circulation unit which causes the microbe flowing out of an end of the flow channel to flow into another end of the flow channel to circulate the microbe.
(11) In a third aspect of the present disclosure, there is provided a method including receiving a plurality of captured images capturing a flow channel through which a microbe flows; and extracting a plurality of microbial images capturing a same individual of the microbe from the plurality of captured images.
(12) In a fourth aspect of the present disclosure, there is provided a program which, when executed by a computer, causes the computer to function as an acquisition unit which receives a plurality of captured images capturing a flow channel through which a microbe flows, and an extraction unit which extracts a plurality of microbial images capturing a same individual of the microbe from the plurality of captured images.

Note that a summary of the invention described above does not necessarily list all features of the present invention. In addition, the present invention may also be a sub-combination of these features.

### BRIEF DESCRIPTION OF THE DRAWINGS

Fig. 1 illustrates a configuration of a system 10 according to the present embodiment along with an input apparatus 40 and an output apparatus 50.
Fig. 2 illustrates a learning process in an identification apparatus 60 according to the present embodiment.
Fig. 3 illustrates an identification process in the identification apparatus 60 according to the present embodiment.
Fig. 4 illustrates an example of image extraction according to the present embodiment.
Fig. 5 illustrates a plan view of a flow channel 500 according to a variant.
Fig. 6 illustrates a longitudinal sectional view of the flow channel 500 according to the variant.
Fig. 7 illustrates an example of a computer 1200 in which a plurality of aspects of the present invention may be entirely or partially embodied.

### DESCRIPTION OF EXEMPLARY EMBODIMENTS

The present invention will be described below by way of embodiments of the invention, but the embodiments below are not intended to limit the invention according to the claims. In addition, not all combinations of features described in the embodiments are necessarily essential to a solution of the invention.

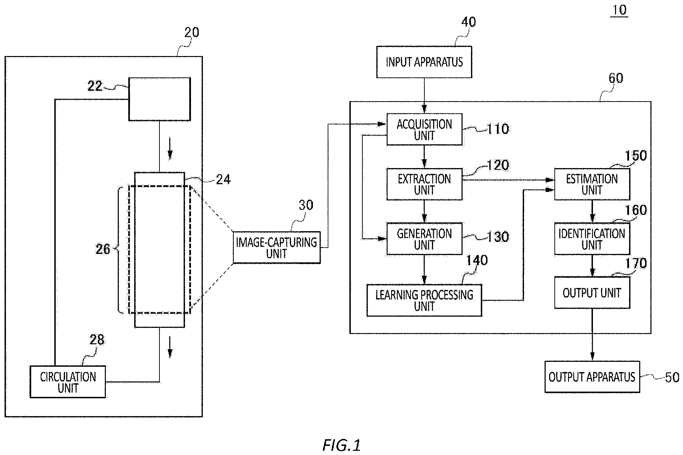

Fig. 1 illustrates a configuration of a system 10 according to the present embodiment, along with an input apparatus 40 and an output apparatus 50. The input apparatus 40 supplies an instruction to the system 10, which is input by a user of the system 10 and the like to the system 10. The output apparatus 50 displays a display screen output by the system 10. The input apparatus 40 and the output apparatus 50 may be implemented by a same computer, terminal apparatus, console apparatus, or the like, or may be implemented by a different computer, terminal apparatus, console apparatus, or the like.

From a plurality of captured images capturing a flow channel through which one or more microbes flow, the system 10 extracts a plurality of microbial images in which a same individual microbe is captured, and generates learning data. In the present embodiment, the microbes may be plankton, and may be at least one of phytoplankton or zooplankton. In addition, the microbes are unicellular or multicellular and may include a virus. The system 10 identifies a species of the microbes from the plurality of microbial images. In an example of the present illustration, the system 10 includes a flow channel apparatus 20, one or more image-capturing units 30, and an identification apparatus 60.

A fluid containing the one or more microbes flows through the flow channel apparatus 20. In the present embodiment, the fluid may be a liquid or water. The flow channel apparatus 20 includes a container 22, a flow channel 24, and a circulation unit 28. Alternatively, the flow channel apparatus 20 may not include the circulation unit 28.

The container 22 stores the fluid containing the one or more microbes. As long as the container 22 can store the fluid, its shape and material are not limited.

The fluid introduced from the container 22 flows through the flow channel 24. As an example, the fluid may flow from an upstream side, which is a top side of a paper in the present illustration, to a downstream side, which is a bottom side of the paper in the present illustration, in the flow channel 24. Since the microbes are pushed by the fluid, they flow rotationally in the flow channel 24. The flow channel 24 may include a wall surface at least partially formed of a transparent material such as glass or plastic. The flow channel 24 may be a pipe with a fixed shape or a flexible tube. The flow channel 24 may have a certain cross-sectional area. The flow channel 24 may have a circular or polygonal cross-section. The flow channel 24 may have a diameter which is larger than a size of the microbes flowing in the flow channel 24. The flow channel 24 may have a diameter of 100 µm to 1 mm. The flow channel 24 includes an image-capturing area 26.

The image-capturing area 26 is an area in the flow channel 24 which is to be captured by the image-capturing units 30. The image-capturing area 26 may be an entire flow channel 24, or a part thereof. When the image-capturing area 26 is a part of the flow channel 24, the image-capturing area 26 may be positioned in any region in the flow channel 24. The image-capturing area 26 may include a wall surface formed of a transparent material such as glass or plastic.

The circulation unit 28 is positioned in a downstream part of the flow channel 24. The circulation unit 28 causes the one or more microbes flowing out of an end of the flow channel 24 to flow into another end of the flow channel 24, thereby circulating the microbes. According to the system 10 with such function, the same individual microbe flows through the image-capturing area 26 of the flow channel 24 multiple times, so that the same individual microbe can be captured increased number of times. The circulation unit 28 may be a pump. In the example of the present illustration, the circulation unit 28 causes the fluid containing the microbes and flowing out of the downstream side of the flow channel 24 to flow into the container 22. Alternatively, the circulation unit 28 may cause the fluid containing the microbes and flowing out of the downstream side of the flow channel 24 to flow into an upstream end of the flow channel 24. When the flow channel apparatus 20 does not include the circulation unit 28, the flow channel apparatus 20 may discharge the fluid containing the microbe and flowing out of the downstream side of the flow channel 24 outside the flow channel apparatus 20.

The one or more image-capturing units 30 capture the plurality of captured images which capture the image-capturing area 26 of the flow channel 24. One image-capturing unit 30 may capture the flow channel 24 multiple times at different timings. When the system 10 includes a plurality of image-capturing units 30, the plurality of image-capturing units 30 may capture the flow channel 24 at different timings from each other. The plurality of image-capturing units 30 may capture the flow channel 24 from different directions. The image-capturing units 30 may be fixed, or provided in a movable manner. The image-capturing units 30 may include an image-capturing apparatus including an image-capturing element, such as a camera, and a control apparatus which controls the image-capturing apparatus. The image-capturing units 30 may further include communication circuitry or input/output interface circuitry.

The identification apparatus 60 may be a computer such as a PC, or personal computer, a tablet computer, a smartphone, a workstation, a server computer, or a general-purpose computer, or may be a computer system in which a plurality of computers are connected. Such a computer system is also a computer in a broad sense. In addition, the identification apparatus 60 may be implemented by a virtual computer environment, one or more of which are executable in a computer. Alternatively, the identification apparatus 60 may be a dedicated computer designed for the system 10, or may be dedicated hardware achieved by dedicated circuitry. The identification apparatus 60 includes an acquisition unit 110, an extraction unit 120, a generation unit 130, a learning processing unit 140, an estimation unit 150, an identification unit 160, and an output unit 170.

The acquisition unit 110 is connected to the image-capturing units 30 and the input apparatus 40. The acquisition unit 110 may further be connected to a storage apparatus, such as a USB, external to the system 10. The acquisition unit 110 may be connected to each of the image-capturing units 30, the input apparatus 40, and the storage apparatus external to the system 10, via various wired or wireless networks such as the Internet, the wide area network, or WAN, the local area network, and the mobile network, or a network such as a combination thereof. The acquisition unit 110 may be connected to each of the image-capturing units 30, the input apparatus 40, and the external storage apparatus via an input/output line. The acquisition unit 110 receives the plurality of captured images from the image-capturing units 30. The acquisition unit 110 receives a user input, an image, and the like, from the input apparatus 40. The acquisition unit 110 may include communication circuitry, input/output interface circuitry, or the like, and may acquire the captured images by receiving the captured images from the image-capturing units 30, using the communication circuitry or the input/output interface circuitry. The acquisition unit 110 may acquire the user input by receiving the user input from the input apparatus 40, using the communication circuitry or the input/output interface circuitry. The acquisition unit 110 may acquire the image by receiving the image from the external storage apparatus, using the communication circuitry or the input/output interface circuitry.

The extraction unit 120 is connected to the acquisition unit 110. The extraction unit 120 receives the plurality of captured images from the acquisition unit 110. From the plurality of captured images received, the extraction unit 120 extracts the plurality of microbial images in which the same individual microbe is captured.

The generation unit 130 is connected to the acquisition unit 110 and the extraction unit 120. The generation unit 130 receives a specified label from the acquisition unit 110. The generation unit 130 receives the plurality of microbial images from the extraction unit 120. The generation unit 130 generates learning data in which the specified label is added to the plurality of microbial images.

The learning processing unit 140 is connected to the generation unit 130. The learning processing unit 140 receives the learning data from the generation unit 130. Using the learning data, the learning processing unit 140 generates an estimation model which estimates the species of the microbe from the microbial images.

The estimation unit 150 is connected to the extraction unit 120 and the learning processing unit 140. In addition to that, the estimation unit 150 may be connected to the acquisition unit 110. The estimation unit 150 receives the plurality of microbial images which have been extracted, from the extraction unit 120. The estimation unit 150 receives the estimation model from the learning processing unit 140. The estimation unit 150 may receive the image input to the input apparatus 40, via the acquisition unit 110. The estimation unit 150 uses each of the plurality of microbial images to estimate the species of the microbe which has been captured.

The identification unit 160 is connected to the estimation unit 150. The identification unit 160 receives an estimation result regarding the species of the microbe in each of the plurality of microbial images, from the estimation unit 150. The identification unit 160 identifies the species of the microbe based on the estimation result regarding the species of the microbe.

The output unit 170 is connected to the identification unit 160 and the output apparatus 50. In addition to that, the output unit 170 may be connected to the extraction unit 120. The output unit 170 receives an identification result from the identification unit 160. The output unit 170 performs a process to have the identification result displayed on a screen of the output apparatus 50, and the like. Here, performing the process to have a screen displayed is not limited to a process to have a screen actually displayed on a display apparatus, but includes a process to generate display data for a screen to be displayed on a remote display apparatus.

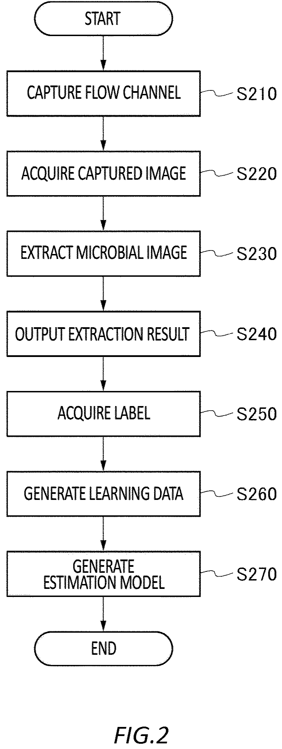

Fig. 2 illustrates a learning process in the identification apparatus 60 according to the present embodiment. In a step 210, or S210, the image-capturing units 30 capture the plurality of captured images which capture the image-capturing area 26 of the flow channel 24 through which the microbes flow. The image-capturing unit 30 may capture at least one of a still image or a moving image. The image-capturing unit 30 may capture the plurality of captured images by capturing still images multiple times. Alternatively, the image-capturing units 30 may capture the plurality of captured images by extracting a plurality of frame images in the moving image with a certain length of time. When a flow velocity of the fluid flowing through the flow channel 24 is v, a length of the image-capturing area 26 is L, and the image-capturing units 30 capture N captured images, the image-capturing units 30 may perform capturing with a capturing interval Δt, which satisfies v × Δt × N ≤ L.

In S220, the acquisition unit 110 receives the plurality of captured images that capture the flow channel through which the microbes flow. The acquisition unit 110 may receive the plurality of captured images from the image-capturing units 30 via a network and the like. The acquisition unit 110 may receive the plurality of captured images which capture the flow channel 24 at different timings. According to the system 10 with such function, it is possible to receive the plurality of captured images in different states, such as an orientation and a position, of the microbes flowing through the flow channel 24. The acquisition unit 110 may receive the plurality of captured images in which the flow channel 24 is captured at different timings by a single image-capturing unit 30. The acquisition unit 110 may receive the captured images in which the flow channel 24 is captured at different timings by each of the plurality of image-capturing units 30. The acquisition unit 110 may receive the plurality of captured images in which the flow channel 24 is captured from two or more different directions. According to the system 10 with such function, it is possible to receive the plurality of captured images with different relative orientations with respect to the microbes, for each of the one or more microbes flowing through the flow channel 24. The acquisition unit 110 may receive the plurality of captured images in which the flow channel 24 is captured from two or more different directions by a movable single image-capturing unit 30. The acquisition unit 110 may receive the captured images captured by each of the plurality of image-capturing units 30 installed in different directions with respect to the flow channel 24.

In S230, the extraction unit 120 receives the plurality of captured images from the acquisition unit 110. The extraction unit 120 extracts the plurality of microbial images in which the same individual microbe is captured, from the plurality of captured images. Here, the microbial images may be an image of a portion of each captured image in which the microbe is captured. The extraction unit 120 may detect a microbial region which includes the microbe entirely, from each of the plurality of captured images. The extraction unit 120 may identify whether the microbe included in each microbial region is the same individual, based on at least one of a positional relationship or similarity among microbial regions in each of the plurality of captured images. The extraction unit 120 may extract an image of each microbial region including the same individual, as the plurality of microbial images in which the same individual microbe is captured. The extraction unit 120 may receive captured images in which a plurality of microbes are captured. The extraction unit 120 may extract a plurality of microbial images in which the same individual microbe is captured, for each of the plurality of microbes.

In S240, the output unit 170 outputs an extraction result by the extraction unit 120. The output unit 170 may output the plurality of microbial images extracted by the extraction unit 120, in which the same individual microbe is captured. Then, the output unit 170 may group and output the plurality of microbial images in which a same microbe is captured. The output unit 170 may output at least some of the plurality of microbial images in which the same microbe is captured.

In S250, the user inputs the label indicating the species of the microbe to the input apparatus 40, based on at least some of the plurality of microbial images displayed by the output apparatus 50, in which the same microbe is captured. The acquisition unit 110 acquires a user-specified label via the input apparatus 40. In the present embodiment, the label may be text data which indicates the species of the microbe captured in the plurality of microbial images output in S240.

In S260, the generation unit 130 acquires the specified label from the acquisition unit 110. The generation unit 130 acquires the plurality of microbial images from the extraction unit 120. The generation unit 130 generates learning data in which the specified label is added to the plurality of microbial images. According to the system 10 with such function, since the label can be added collectively to the plurality of microbial images, not individually to each of the microbial images, it is possible to reduce a workload for labelling. According to the system 10 with such function, since a same label can be added to the plurality of microbial images, a labelling process can be accelerated. According to the system 10 with such function, since it is possible to capture the microbes multiple times while they are flowing through the flow channel 24 and add the label collectively thereto, the learning data can be generated efficiently. The generation unit 130 may have the generated learning data to be stored in a storage apparatus and the like, external to the identification apparatus 60.

In S270, the learning processing unit 140 acquires the learning data from the generation unit 130. Using the learning data, the learning processing unit 140 performs a process to generate the estimation model through learning, which estimates the species of the microbe, from the microbial images. The learning processing unit 140 may use the learning data to generate a new estimation model. The learning processing unit 140 may use the learning data to update an existing estimation model, thereby generating the estimation model. The learning processing unit 140 may use a machine-learning algorithm, such as a neural network and a convolutional neural network, to generate the estimation model. According to the system 10 with such function, it is possible to generate the estimation model which can estimate the microbe regardless of the direction in which the microbe is captured. According to the system 10 with such function, since it is possible to use the efficiently generated learning data for learning, the estimation accuracy can be improved by using more learning data for learning. When the plurality of captured images, which capture the microbes circulated by the circulation unit 28, are used as the learning data, the system 10 can use more learning data for each of the microbes, so that the estimation accuracy can be improved.

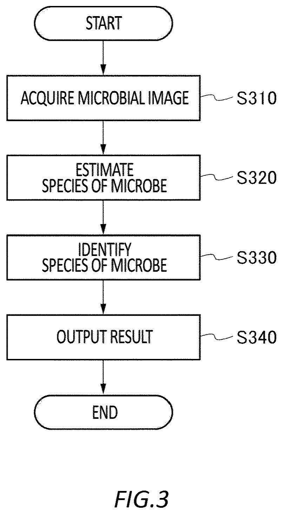

Fig. 3 illustrates an identification process in the identification apparatus 60 according to the present embodiment. In S310, the acquisition unit 110 acquires one or more images which capture the microbe to be identified. The acquisition unit 110 may acquire, from the image-capturing units 30, the captured images capturing the flow channel 24 through which the microbes flow, as images capturing the microbe to be identified. The acquisition unit 110 may acquire one microbial image capturing the microbe to be identified from the image-capturing units 30. When the flow velocity of the fluid flowing through the flow channel 24 is v, and the length of the image-capturing area 26 is L, the image-capturing units 30 may perform capturing with the capturing interval Δt, which satisfies v × Δt ≥ L. Alternatively, the acquisition unit 110 may acquire a plurality of images capturing the microbe to be identified from the image-capturing units 30. When the image-capturing units 30 capture N captured images, the image-capturing units 30 may perform capturing with the capturing interval Δt, which satisfies v × Δt × N ≤ L. When the acquisition unit 110 acquires the plurality of images from the image-capturing units 30, the extraction unit 120 may use a similar method as S230 in Fig. 2 to extract the plurality of microbial images in which the same individual microbe is captured, from the plurality of captured images. The acquisition unit 110 may acquire an image capturing the microbe to be identified from the input apparatus 40.

In S320, the estimation unit 150 may acquire one or more microbial images from the acquisition unit 110. Alternatively, the estimation unit 150 may acquire the plurality of microbial images in which the same individual microbe is captured, from the extraction unit 120. The estimation unit 150 may use the estimation model to estimate the species of the microbe. The estimation unit 150 may acquire the estimation model from the learning processing unit 140. The estimation unit 150 may acquire the estimation model from outside the system 10 via the acquisition unit 110. The estimation unit 150 uses each of the one or more microbial images to estimate the species of the microbe. When the estimation unit 150 uses one microbial image to estimate the species of the microbe based on the estimation model acquired from the learning processing unit 140, since it uses a model which has learnt microbial images captured from various directions, estimation can be performed with high accuracy even when there is only one image which captures the microbe to be identified. In addition, since what is required is only one image which captures the microbe to be identified, the system 10 can perform the estimation quickly. When the estimation unit 150 uses each of the plurality of microbial images extracted by the extraction unit 120 to estimate the species of the microbe, the microbial images captured from the various directions are used, so that the estimation can be performed with higher accuracy.

In S330, the identification unit 160 may identify the species of the microbe, based on the estimation result regarding the species of the microbe estimated by using each of the one or more microbial images. When the estimation unit 150 estimates the species of the microbe for one microbial image, the identification unit 160 may identify the species of the microbe estimated by the estimation unit 150 as the species of that microbe. In this case, the system 10 can reduce time required for identification. When the identification unit 160 identifies the species of the microbe based on a plurality of estimation results, determination accuracy can be improved. When the estimation unit 150 estimates the species of the microbe for each of the plurality of microbial images in which the same individual microbe is captured, the identification unit 160 may identify the species of the microbe estimated in most images by the estimation unit 150 as the species of the microbe of that individual. When the estimation unit 150 estimates the species of the microbe for each of the plurality of microbial images in which the same individual microbe is captured, the identification unit 160 may identify the species of the microbe based on an estimation result with highest reliability among the estimation results made by the estimation unit 150. For example, the identification unit 160 may identify the species of the microbe estimated in a microbial image with highest similarity with the learning data, as the species of that microbe.

In S340, the output unit 170 outputs a determination result made by the identification unit 160. The output unit 170 may output the species of all microbes included in the images acquired by the acquisition unit 110 in S310. The output unit 170 may output the species and the numbers of all microbes included in the images acquired by the acquisition unit 110 in S310. When the acquisition unit 110 acquires, in S310, the plurality of captured images captured by the image-capturing units 30 within a certain length of time, the output unit 170 may output the species of the microbes, or the species and the numbers of the microbes flowing through the image-capturing area 26 within the certain length of time.

In S240 of Fig. 2, the output unit 170 may output the plurality of microbial images capturing the same individual microbe, and the species of that individual identified by the estimation unit 150 and the identification unit 160. In this case, the estimation unit 150 may estimate the species of the microbe with a method illustrated in S320 of Fig. 3. The identification unit 160 may identify the species of the microbe with a method illustrated in S330 of Fig. 3.

According to the system 10 illustrated above, it is possible to acquire the microbial images captured from the various directions in a short time with little effort.

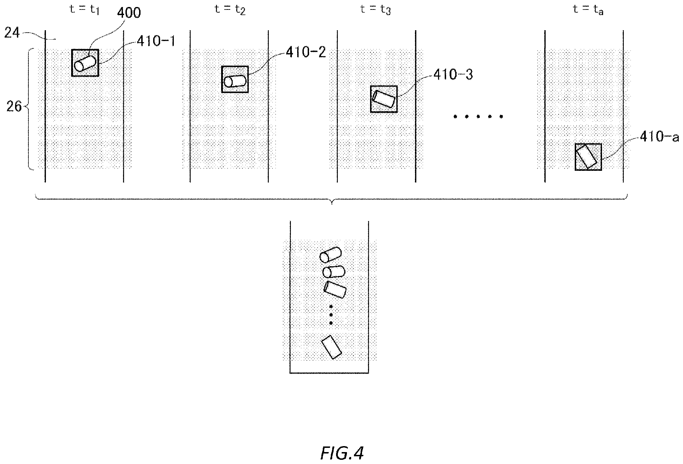

Fig. 4 illustrates an example of image extraction according to the present embodiment. In S230 of Fig. 2, the extraction unit 120 acquires the plurality of captured images. In the example of the present illustration, the extraction unit 120 acquires the plurality of captured images which capture the image-capturing area 26 of the flow channel 24 through which plankton 400 flows, at each of capturing times t = t₁, t₂, t₃, ... tₙ. The extraction unit 120 detects a microbial region 410 containing the plankton 400, such as a microbial region 410-1, 410-2, 410-3, ... 410-a, in each of the plurality of captured images. The extraction unit 120 may divide each captured image into a plurality of blocks. The extraction unit 120 may divide each captured image into a plurality of square or rectangular blocks. The extraction unit 120 determines whether or not each block contains at least part of the plankton 400. The extraction unit 120 may determine whether or not each block contains at least part of the plankton 400, based on a hue, lightness, brightness, and the like of each block. The extraction unit 120 detects the microbial region 410 containing the plankton 400, based on a collection of blocks determined to contain at least part of the plankton 400. The extraction unit 120 may perform edge detection in each of the plurality of captured images to detect a region including detected edges as the microbial region 410. The microbial region 410 may be a square or rectangular region. Although the extraction unit 120 detects one microbial region 410 in one captured image in the example of the present illustration, it may detect a plurality of microbial regions 410 in one captured image.

The extraction unit 120 identifies whether the plankton 400 contained in each microbial region 410 is the same individual. The extraction unit 120 may identify whether the plankton 400 is the same individual, based on the positional relationship between microbial regions 410 in respective captured images. In the example of the present illustration, the extraction unit 120 calculates the positional relationship between the microbial region 410-1 and the microbial region 410-2. The extraction unit 120 may calculate a distance from the microbial region 410-1 to the microbial region 410-2, such as a distance between center points, a distance between upper ends, or a distance between lower ends. The extraction unit 120 may compare the distance from the microbial region 410-1 to the microbial region 410-2 and a moving distance of the fluid calculated based on the flow velocity of the fluid and an interval between the capturing times. When a difference between the distance from the microbial region 410-1 to the microbial region 410-2 and the moving distance of the fluid is below a threshold, the extraction unit 120 may determine that the plankton 400 contained in the microbial region 410-1 and the plankton 400 contained in the microbial region 410-2 are the same individual.

The extraction unit 120 may identify whether the plankton 400 is the same individual, based on the similarity between the microbial regions 410 in respective captured images. The extraction unit 120 may calculate the similarity between the microbial regions 410 based on a hue, lightness, brightness, and the like of the microbial regions 410 in respective captured images. The extraction unit 120 may calculate the similarity between the microbial regions 410 based on a shape of the plankton 400 contained in each of the microbial regions 410. When the similarity between the microbial regions 410 is above a threshold, the extraction unit 120 may determine that the plankton 400 contained in respective microbial regions 410 is the same individual. When the difference between the distance between the microbial regions 410 and the moving distance of the fluid is below the threshold and the similarity between the microbial regions 410 is above the threshold, the extraction unit 120 may determine that the plankton 400 contained in respective microbial regions 410 is the same individual. When differences between a distance from either of a microbial region A and a microbial region B, which are two microbial regions 410 at a certain time, to a microbial region 410 at a different time, and the moving distance of the fluid are both below the threshold, the extraction unit 120 may identify whether the plankton 400 is the same individual based on the similarity between the microbial regions 410.

The extraction unit 120 extracts the plurality of microbial images capturing a same individual plankton 400. The extraction unit 120 may extract the plurality of microbial regions 410 containing the same individual plankton 400 in the plurality of captured images, as the plurality of microbial images. The extraction unit 120 may extract the plurality of microbial regions 410 containing the same individual plankton 400 as a set of images. As illustrated in a bottom part of the present illustration, the extraction unit 120 may integrate the plurality of microbial regions 410 containing the same individual plankton 400 to generate one image including a plurality of images capturing the same individual plankton 400.

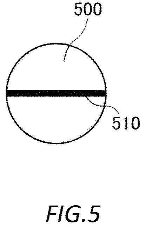

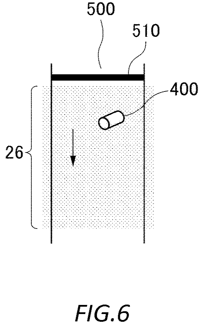

Fig. 5 illustrates a plan view of a flow channel 500 according to a variant. Fig. 6 illustrates a longitudinal sectional view of the flow channel 500 according to the variant. In an example of the present illustration, the flow channel 500 includes the image-capturing area 26 and a vortex generator 510. The flow channel 500 according to the variant may be similar to the flow channel 24 of Fig. 1, except that it includes the vortex generator 510.

The vortex generator 510 generates a vortex in the image-capturing area 26 in the flow channel 24, in which the plurality of captured images are captured. The vortex generator 510 may be positioned in the fluid flowing through the flow channel 24. The vortex generator 510 may be positioned upstream or downstream from the image-capturing area 26. The vortex generator 510 may be an object with a particular shape. In the example of the present illustration, the vortex generator 510 is an object with a cuboid shape, which is positioned at a diameter of a transverse cross-section of the flow channel 500. The vortex generator 510 may be an object with a shape of a triangular prism, cylinder, or trapezoid. Alternatively, the vortex generator 510 may be a protrusion formed on an inner wall surface of the flow channel 500. The vortex generator 510 may be a helical protrusion formed on the inner wall surface. Alternatively, the vortex generator 510 may be a stirring element. Alternatively, the vortex generator 510 may generate a vortex by rotating a wall surface of the flow channel 24. According to the system 10 with such vortex generator 510, since orientations of the microbes flowing through the image-capturing area 26 are more likely to change, captured images with different relative orientations with respect to the microbes can be acquired more easily.

Various embodiments of the present invention may be described with reference to a flowchart and a block diagram whose block may represent (1) a stage of a process in which an operation is executed or (2) a section of an apparatus responsible for executing the operation. A particular stage and section may be implemented by a dedicated circuit, a programmable circuit supplied together with a computer-readable instruction stored on a computer-readable medium, and/or a processor supplied together with the computer-readable instruction stored on the computer-readable medium. The dedicated circuit may include a digital and/or analog hardware circuit and may include an integrated circuit, or IC, and/or a discrete circuit. The programmable circuit may include a reconfigurable hardware circuit including logical AND, logical OR, logical XOR, logical NAND, logical NOR, and another logical operation, a memory element or the like such as a flip-flop, a register, a field programmable gate array, or FPGA, a programmable logic array, or PLA, or the like.

The computer-readable medium may include any tangible device that can store an instruction to be executed by an appropriate device, and as a result, the computer-readable medium including an instruction stored thereon will include a product including the instruction that may be executed to create means for executing the operation specified in the flowchart or the block diagram. Examples of the computer-readable medium may include an electronic storage medium, a magnetic storage medium, an optical storage medium, an electromagnetic storage medium, a semiconductor storage medium, or the like. More specific examples of the computer-readable medium may include a floppy (registered trademark) disk, a diskette, a hard disk, a random access memory, or RAM, a read-only memory, or ROM, an erasable programmable read-only memory, or EPROM or flash memory, an electrically erasable programmable read-only memory, or EEPROM, a static random access memory, or SRAM, a compact disc read-only memory, or CD-ROM, a digital versatile disk, or DVD, a Blu-ray (registered trademark) disk, a memory stick, an integrated circuit card, or the like.

The computer-readable instruction may include an assembler instruction, an instruction-set-architecture, or ISA instruction, a machine instruction, a machine-dependent instruction, a microcode, a firmware instruction, state-setting data, or either a source code or an object code described in any combination of one or more programming languages, including an object-oriented programming language such as Smalltalk (registered trademark), JAVA (registered trademark), C++, or the like, and a conventional procedural programming language such as a "C" programming language or a similar programming language.

The computer-readable instruction may be provided for a processor or programmable circuit o a programmable data processing apparatus, such as a computer, locally or via a local area network (LAN), a wide area network (WAN) such as the Internet, or the like to execute the computer-readable instruction to create means for executing the operations specified in the flowcharts or block diagrams. Here, the computer may be a personal computer, or PC, a tablet computer, a smartphone, a workstation, a server computer, a general-purpose computer, a special-purpose computer, or the like, or may be a computer system to which a plurality of computers are connected. Such computer system to which the plurality of computers are connected is also referred to as a distributed computing system, and is a computer in a broad sense. In a distributed computing system, a plurality of computers collectively execute a program by each of the plurality of computers executing a part of the program, and passing data during the execution of the program among the computers as needed.

Examples of the processor include a computer processor, a central processing unit (CPU), a processing unit, a microprocessor, a digital signal processor, a controller, a microcontroller, and the like. The computer may include one processor or a plurality of processors. In a multi-processor system including a plurality of processors, the plurality of processors collectively execute a program by each of the processors executing a part of the program, and passing data during the execution of the program among the processors as needed. For example, in execution of multiple tasks, each of the plurality of processors may execute a part of each task pieces by pieces by performing task-switching for each time slice. In this case, which part of one program each processor is responsible for executing dynamically changes. Moreover, which part of the program each of the plurality of processors is responsible for executing may be determined statically by multi-processor-aware programming.

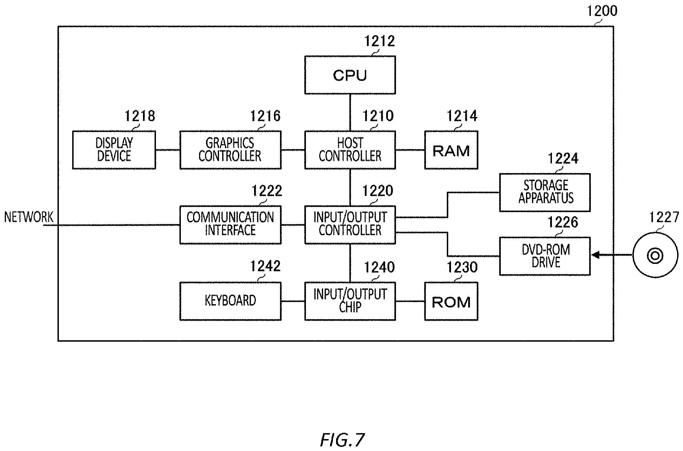

Fig. 7 illustrates an example of a computer 1200 in which a plurality of aspects of the present invention may be entirely or partially embodied. A program that is installed in the computer 1200 may cause the computer 1200 to function as operations associated with an apparatus according to the embodiment of the present invention or one or more sections in the apparatus, or may cause the computer 1200 to execute the operation or the one or more sections, and/or may cause the computer 1200 to execute processes according to the embodiment of the present invention or stages of the processes. Such a program may be executed by a CPU 1212 in order to cause the computer 1200 to execute particular operations associated with some or all of the blocks of flowcharts and block diagrams described herein.

The computer 1200 according to the present embodiment includes a CPU 1212, a RAM 1214, a graphics controller 1216, and a display device 1218, which are mutually connected by a host controller 1210. The computer 1200 also includes a communication interface 1222, a storage apparatus 1224 such as a hard disk, input/output units such as a DVD-ROM drive 1226 and an IC card drive, which are connected to the host controller 1210 via an input/output controller 1220. The computer also includes legacy input/output units such as an ROM 1230 and a keyboard 1242, which are connected to the input/output controller 1220 via an input/output chip 1240.

The CPU 1212 operates according to programs stored in the ROM 1230 and the RAM 1214, thereby controlling each unit. The graphics controller 1216 acquires image data generated by the CPU 1212 on a frame buffer or the like provided in the RAM 1214 or in itself, and causes the image data to be displayed on a display device 1218.

The communication interface 1222 communicates with another electronic device via a network. The storage apparatus 1224 stores a program and data used by the CPU 1212 in the computer 1200. The DVD-ROM drive 1226 reads a program or data from a DVD-ROM 1227 and provides the program or data to the storage apparatus 1224 via the RAM 1214. The IC card drive reads the programs and the data from the IC card, and/or writes the programs and the data to the IC card.

The ROM 1230 stores therein a boot program or the like that is executed by the computer 1200 at the time of activation, and/or a program which depends on the hardware of the computer 1200. The input/output chip 1240 may also connect various input/output units to the input/output controller 1220 via a parallel port, a serial port, a keyboard port, a mouse port, or the like.

Programs are provided by a computer-readable medium such as the DVD-ROM 1227 or the IC card. The programs are read from the computer-readable medium, are installed in the storage apparatus 1224, the RAM 1214, or the ROM 1230, which are also an example of the computer-readable medium, and are executed by the CPU 1212. Information processing written in these programs is read by the computer 1200, and provides cooperation between the programs and the various types of hardware resources described above. An apparatus or method may be constructed by realizing the operation or processing of information according to the use of the computer 1200.

For example, when communication is executed between the computer 1200 and an external device, the CPU 1212 may execute a communication program loaded onto the RAM 1214 to instruct communication processing to the communication interface 1222, based on the processing described in the communication program. Under the control of the CPU 1212, the communication interface 1222 reads transmission data stored in a transmission buffer processing region provided in a recording medium such as the RAM 1214, the storage apparatus 1224, the DVD-ROM 1227, or the IC card, transmits the read transmission data to the network, or writes reception data received from the network in a reception buffer processing region or the like provided on the recording medium.

In addition, the CPU 1212 may cause the RAM 1214 to read all or a necessary portion of a file or database stored in an external recording medium such as the storage apparatus 1224, the DVD-ROM drive 1226, or the DVD-ROM 1227, the IC card, or the like, and may execute various types of processes on data on the RAM 1214. The CPU 1212 may then write back the processed data to the external recording medium.

Various types of information such as various types of programs, data, tables, and databases may be stored in a recording medium and subjected to information processing. The CPU 1212 may execute various types of processing on the data read from the RAM 1214, which includes various types of operations, information processing, conditional judging, conditional branch, unconditional branch, search/replace of information, or the like, as described throughout the present disclosure and specified by an instruction sequence of programs, and writes the result back to the RAM 1214. In addition, the CPU 1212 may search for information in a file, a database, or the like in the recording medium. For example, when a plurality of entries, each having an attribute value of a first attribute associated with an attribute value of a second attribute, are stored in the recording medium, the CPU 1212 may search, out of the plurality of entries, an entry with the attribute value of the first attribute specified that matches a condition, read the attribute value of the second attribute stored in said entry, and thereby acquiring the attribute value of the second attribute associated with the first attribute satisfying a predetermined condition.

The above-described program or software module may be stored in the computer-readable medium on the computer 1200 or near the computer 1200. In addition, a recording medium such as a hard disk or a RAM provided in a server system connected to a dedicated communication network or the Internet may be used as the computer-readable medium, thereby providing the program to the computer 1200 via the network.

While the present invention has been described above by way of the embodiments, the technical scope of the present invention is not limited to the scope described in the above-described embodiments. It is apparent to persons skilled in the art that various changes or improvements can be made to the above-described embodiments. It is also apparent from description of the claims that the embodiments to which such changes or improvements are made may be included in the technical scope of the present invention.

It should be noted that each process of the operations, procedures, steps, stages, and the like performed by the apparatus, system, program, and method shown in the claims, specification, and drawings may be executed in any order as long as the order is not particularly explicitly indicated by "prior to", "before", or the like and as long as an output from a previous process is not used in a later process. Even when the operational flow in the claims, specification, and drawings is described using phrases such as "first", "next", or the like for the sake of convenience, it does not necessarily mean that the process must be performed in this order.

### EXPLANATION OF REFERENCES

10: system; 20: flow channel apparatus; 22: container; 24: flow channel; 26: image-capturing area; 28: circulation unit; 30: image-capturing unit; 40: input apparatus; 50: output apparatus; 60: identification apparatus; 110: acquisition unit; 120: extraction unit; 130: generation unit; 140: learning processing unit; 150: estimation unit; 160: identification unit; 170: output unit; 400: plankton; 410: microbial region; 500: flow channel; 510: vortex generator; 1200: computer; 1210: host controller; 1212: CPU; 1214: RAM; 1216: graphics controller; 1218: display device; 1220: input/output controller; 1222: communication interface; 1224: storage apparatus; 1226: DVD-ROM drive; 1227: DVD-ROM; 1230: ROM; 1240: input/output chip; 1242: keyboard.

## Claims

1. An apparatus comprising a processor, wherein
the processor
receives a plurality of captured images capturing a flow channel through which a microbe flows, and
extracts a plurality of microbial images capturing a same individual of the microbe from the plurality of captured images.

2. The apparatus according to claim 1, wherein
the flow channel comprises a vortex generator which generates a vortex in an image-capturing area in the flow channel, in which the plurality of captured images are captured.

3. The apparatus according to claim 1 or 2, wherein
in receiving the plurality of captured images, the processor receives the plurality of captured images which capture the flow channel at different timings.

4. The apparatus according to any one of claims 1 to 3, wherein
in receiving the plurality of captured images, the processor receives the plurality of captured images which capture the flow channel from two or more different directions.

5. The apparatus according to any one of claims 1 to 4, wherein
the processor further generates learning data in which a specified label is added to the plurality of microbial images.

6. The apparatus according to claim 5, wherein
the processor further performs a process to generate an estimation model through learning, which estimates a species of the microbe from the microbial images, using the learning data.

7. The apparatus according to any one of claims 1 to 6, wherein
the processor uses each of the plurality of microbial images to further estimate a species of the microbe.

8. The apparatus according to claim 7, wherein
the processor further identifies the species of the microbe, based on an estimation result regarding the species of the microbe estimated by using each of the plurality of microbial images.

9. A system comprising:
the apparatus according to any one of claims 1 to 8;
the flow channel; and
an image-capturing unit which captures the plurality of captured images.

10. The system according to claim 9, further comprising:
a circulation unit which causes the microbe flowing out of an end of the flow channel to flow into another end of the flow channel to circulate the microbe.

11. A method comprising:
receiving a plurality of captured images capturing a flow channel through which a microbe flows; and
extracting a plurality of microbial images capturing a same individual of the microbe from the plurality of captured images.

12. A program which, when executed by a computer, causes the computer to function as
an acquisition unit which receives a plurality of captured images capturing a flow channel through which a microbe flows, and
an extraction unit which extracts a plurality of microbial images capturing a same individual of the microbe from the plurality of captured images.
